# EUROPEAN PATENT APPLICATION

(11) **EP 4 501 370 A1**
(43) Date of publication of application: **05.02.2025**
(21) Application number: 23830933.0
(22) Date of filing: 29.05.2023
(51) Int. Cl.: A61M 1/36

(54) **BLOOD PURIFICATION DEVICE**

(30) Priority: 28.06.2022 JP 2022103784
(71) Applicant: Nikkiso Co., Ltd., Tokyo 150-6022 (JP)
(72) Inventor: SHIGETA, Shinya, Makinohara-shi Shizuoka 421-0496 (JP)
(74) Representative: von Hirschhausen, Helge
(86) International application number: PCT/JP2023/019910
(87) International publication number: WO 2024/004483

(57) **Abstract**

This blood purification device is provided with: a blood circuit (21) for circulating blood through a dialyzer (10) having a blood purification membrane built therein; a dialysis solution supply flow channel (52) for supplying a dialysis solution to the dialyzer (10); an effluent discharge flow channel (53) for discharging effluent from the dialyzer (10); a liquid feeding pump (61) that is disposed to the dialysis solution supply flow channel (52) and that applies pressure to the dialysis solution supplied to the dialyzer (10); and an effluent pump (71) that is disposed to the effluent discharge flow channel (53), that can be driven independently and separately from the liquid feeding pump (61), and that applies pressure to the effluent discharged from the dialyzer (10). By driving the effluent pump (71) in a state where the blood circuit (21) and the effluent discharge flow channel (53) are connected, liquid inside the blood circuit (21) is suctioned to the effluent discharge flow channel (53).

## Description

### TECHNICAL FIELD

The present invention relates to a blood purification device.

### BACKGROUND OF THE INVENTION

There is a blood purification device that uses a water removal pump to prime the blood circuit or to discharge a replacement solution present in the blood circuit (see Patent Literature 1). This blood purification device includes a blood circuit capable of extracorporeally circulating blood, a dialyzer to purify blood flowing through the blood circuit, a tube unit including a dialysate introduction line to introduce dialysate into the dialyzer and a dialysate discharge line to discharge dialysate from the dialyzer, a duplex pump arranged on the dialysate introduction line as well as on the dialysate discharge line to supply dialysate to the dialyzer and discharge effluent from the dialyzer, a water removal pump arranged on a bypass line bypassing the duplex pump to perform water removal by removing water from blood flowing through the dialyzer, and a connecting line connecting the blood circuit to the dialysate discharge line. In this blood purification device, when priming the blood circuit or discharging the replacement solution present in the blood circuit, the blood circuit is suctioned by driving the water removal pump in a state in which the blood circuit and the dialysate discharge line are connected through the connecting line, thereby performing priming or liquid discharge. In this way, by using the water removal pump to suction the blood circuit, there is no need to separately provide a pump for priming or liquid discharge, and priming and liquid discharge can be performed with a simple configuration.

### CITATION LIST

### PATENT LITERATURES

Patent Literature 1: Japanese Patent No. 6488048

### SUMMARY OF THE INVENTION

The conventional blood purification device uses a water removal pump to suction the blood circuit, hence, there is a limit to increasing the speed of priming or discharge of the replacement solution due to the balance with the pressure (suction force) of the water removal pump. However, there is a demand for more rapid priming and discharge of the replacement solution.

Therefore, it is an object of the invention to provide a blood purification device which has a simple configuration and also is capable of quickly performing priming or discharge of a replacement solution.

A blood purification device in an embodiment of the invention is a blood purification device to purify blood of a patient through a blood purifier having a blood purification membrane therein and comprises:
a blood circuit to circulate the blood through the blood purifier;
a dialysate supply flow path to supply dialysate to the blood purifier;
an effluent discharge flow path to discharge effluent from the blood purifier;
a liquid supply pump arranged on the dialysate supply flow path to cause pressure for supply to the blood purifier to be applied to the dialysate; and
a liquid discharge pump that is arranged on the effluent discharge flow path, is provided separately and can be driven independently from the liquid supply pump, and causes pressure for discharge from the blood purifier to be applied to the effluent,
wherein a liquid in the blood circuit is suctioned into the effluent discharge flow path by driving the liquid discharge pump in a state in which the blood circuit and the effluent discharge flow path are connected in a manner that allows for liquid feeding through a connecting flow path connecting the blood circuit to the effluent discharge flow path while bypassing the blood purifier, or through the blood purifier.

### Advantageous Effects of the Invention

According to an embodiment of the invention, the configuration is simple and also priming or discharge of a replacement solution can be performed quickly.

### BRIEF DESCRIPTION OF DRAWINGS

Fig. 1 is a schematic structural diagram illustrating a structure of a blood purification device in an embodiment of the present invention.
Fig. 2A is a first explanatory diagram illustrating a priming operation.
Fig. 2B is a second explanatory diagram illustrating the priming operation.
Fig. 2C is a third explanatory diagram illustrating the priming operation.
Fig. 3 is a flowchart showing the priming operation.
Fig. 4A is a first explanatory diagram illustrating a replacement solution discharge operation.
Fig. 4B is a second explanatory diagram illustrating the replacement solution discharge operation.
Fig. 5 is a flowchart showing the replacement solution discharge operation.
Fig. 6 is a schematic structural diagram illustrating a structure of the blood purification device in a modification.

### DETAILED DESCRIPTION OF THE INVENTION

A blood purification device in an embodiment of the invention will be described below in reference to the appended drawings. This blood purification device is a medical device that performs dialysis treatment to purify patient's blood using a dialyzer, and is a so-called hemodialysis machine. In particular, this blood purification device uses the liquid discharge pump to prime the blood circuit and discharge the replacement solution present in the blood circuit, allowing priming and discharge of the replacement solution to be performed with a simple configuration and also quickly.

### Configuration of Blood purification device

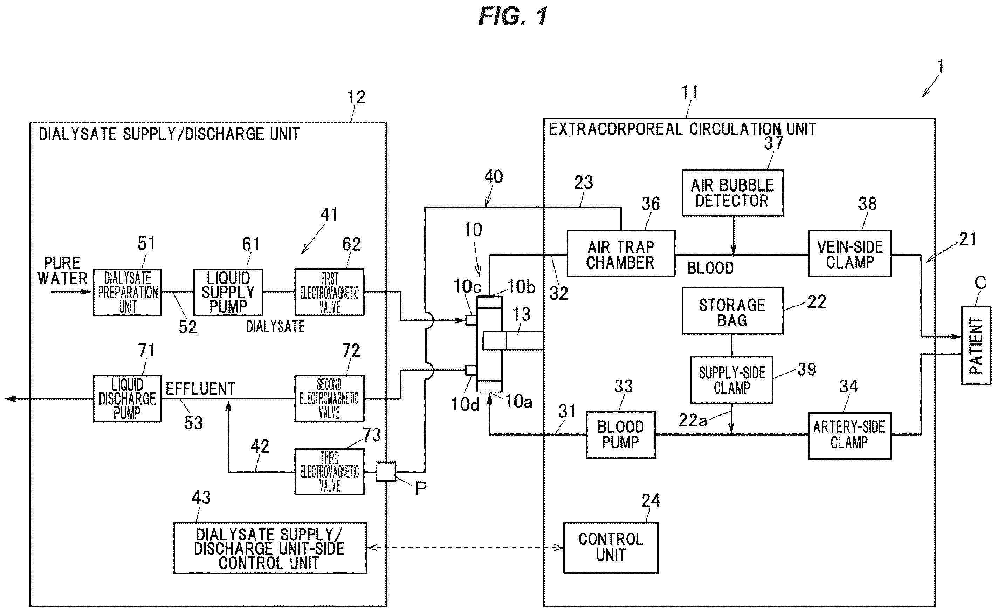

As shown in Fig. 1, a blood purification device 1 includes a dialyzer 10 that purifies blood of a patient C, an extracorporeal circulation unit 11 that circulates blood of the patient C through the dialyzer 10, and a dialysate supply/discharge unit 12 that is connected to the dialyzer 10, supplies dialysate to the dialyzer 10 and discharges effluent from the dialyzer 10. The extracorporeal circulation unit 11 and the dialysate supply/discharge unit 12 are configured as separate units, and the dialyzer 10 is removably attached to the extracorporeal circulation unit 11 through a fixing tool 13. The dialyzer 10 is an example of the blood purifier.

The dialyzer 10 has a blood purification membrane (a hollow-fiber hemodialysis membrane or hemodialysis filtration membrane, or a flat hemodialysis membrane or hemofiltration membrane) thereinside. The dialyzer 10 also has a blood inlet 10a to introduce blood and a blood outlet 10b to discharge the introduced blood, as well as a dialysate inlet 10c to introduce dialysate and a dialysate outlet 10d to discharge the introduced dialysate. In the dialyzer 10, blood is purified by bringing the blood into contact with dialysate through the blood purification membrane.

The extracorporeal circulation unit 11 has a blood circuit 21 to circulate the blood of the patient C through the dialyzer 10, a storage bag 22 connected to the blood circuit 21 through a supply tube 22a, a blood circuit-side connecting tube 23 connected to the blood circuit 21, and a control unit 24. The storage bag 22 is an example of a storage unit that stores a priming solution. The control unit 24 will be described later.

The blood circuit 21 has an artery-side blood tube 31 that is connected to the blood inlet 10a of the dialyzer 10 and leads the blood collected from a blood vessel of the patient C to the dialyzer 10, and a vein-side blood tube 32 that is connected to the blood outlet 10b of the dialyzer 10 and returns the blood discharged from the dialyzer 10 to the blood vessel of the patient C.

A blood pump 33 and an artery-side clamp 34 are provided on the artery-side blood tube 31. The blood pump 33 is a liquid feed pump to feed blood by applying pressure to the blood, and is composed of, e.g., a peristaltic pump. The artery-side 34 is arranged on the upstream side of the blood pump 33 and opens and closes the artery-side blood tube 31.

Meanwhile, an air trap chamber 36, an air bubble detector 37 and a vein-side clamp 38 are arranged on the vein-side blood tube 32. The air trap chamber 36 is a chamber to trap air bubbles in blood. The air bubble detector 37 detects air bubbles in blood. The vein-side clamp 38 is arranged on the downstream side of the air trap chamber 36 and the air bubble detector 37 and opens and closes the vein-side blood tube 32.

In the blood circuit 21, the blood from the patient C is led to the dialyzer 10 through the artery-side blood tube 31 by driving the blood pump 33 in a state in which the artery-side clamp 34 and the vein-side clamp 38 are opened, and the blood is purified by the dialyzer 10 and is then returned to the patient C through the vein-side blood tube 32. The blood of the patient C is thereby purified.

The storage bag 22 is connected through the supply tube 22a to the artery-side blood tube 31 between the blood pump 33 and the artery-side clamp 34. The storage bag 22 stores a saline solution as a priming solution, and during priming, the blood circuit 21 is filled with the saline solution contained in the storage bag 22 through the supply tube 22a. Then, a supply-side clamp 39 to open and close the supply tube 22a is provided on the supply tube 22a.

The blood circuit-side connecting tube 23 is connected to the air trap chamber 36. The blood circuit-side connecting tube 23 is connected to a dialysate circuit-side connecting tube 42 (described later) through a liquid discharge port P and, together with the dialysate circuit-side connecting tube 42, constitutes a connecting flow path 40 that connects the blood circuit 21 to a dialysate circuit 41 (an effluent discharge flow path 53 described later).

The dialysate supply/discharge unit 12 has the dialysate circuit 41 that supplies dialysate to the dialyzer 10 and also discharges effluent from the dialyzer 10, the dialysate circuit-side connecting tube 42 connected to the dialysate circuit 41, and a dialysate supply/discharge unit-side control unit 43.

The dialysate circuit 41 has a dialysate preparation unit 51 that refines dialysate, a dialysate supply flow path 52 that is connected to the dialysate inlet 10c of the dialyzer 10 and supplies the dialysate refined by the dialysate preparation unit 51 to the dialyzer 10, and the effluent discharge flow path 53 that is connected to the dialysate outlet 10d of the dialyzer 10 and collects and discharges effluent from the dialyzer 10.

The dialysate preparation unit 51 prepares dialysate from pure water supplied thereto and a dialysate agent made of a concentrated solution or powder. The pure water supplied to the dialysate preparation unit 51 may be supplied from a pure water production unit mounted on the dialysate supply/discharge unit 12, or may be supplied from a pure water production device provided outside the dialysate supply/discharge unit 12. In this regard, the dialysate preparation unit 51 can be omitted, and the configuration may be such that, e.g., dialysate is supplied to the dialysate supply/discharge unit 12 from an external dialysate supply device, etc.

A liquid supply pump 61 and a first electromagnetic valve 62 are arranged on the dialysate supply flow path 52. The liquid supply pump 61 is a liquid feed pump to feed dialysate by causing pressure for supply to the dialyzer 10 to be applied to the dialysate and is composed of, e.g., a diaphragm pump. The dialysate is supplied to the dialyzer 10 by driving the liquid supply pump 61. The first electromagnetic valve 62 is arranged on the downstream side of the liquid supply pump 61 and opens and closes the dialysate supply flow path 52.

A liquid discharge pump 71 and a second electromagnetic valve 72 are arranged on the effluent discharge flow path 53. The liquid discharge pump 71 is a liquid feed pump that is provided separately and can be driven independently from the liquid supply pump 61 and feeds effluent by causing pressure for discharge from the dialyzer 10 to be applied to the effluent, and is composed of, e.g., a diaphragm pump. The effluent from the dialyzer 10 is discharged by driving the liquid discharge pump 71. The second electromagnetic valve 72 is arranged on the upstream side of the liquid discharge pump 71 and opens and closes the effluent discharge flow path 53.

The dialysate circuit-side connecting tube 42 is connected to the effluent discharge flow path 53 between the liquid discharge pump 71 and the second electromagnetic valve 72. The dialysate circuit-side connecting tube 42 is connected to the blood circuit-side connecting tube 23 through the liquid discharge port P and, together with the blood circuit-side connecting tube 23, constitutes the connecting flow path 40 that connects the blood circuit 21 to the effluent discharge flow path 53 while bypassing the dialyzer 10. That is, the connecting flow path 40 is connected at one end to the air trap chamber 36 on the vein-side blood tube 32 and is connected at the other end to the effluent discharge flow path 53 on the upstream side of the liquid discharge pump 71. A third electromagnetic valve 73 to open and close the connecting flow path 40 is arranged on the dialysate circuit-side connecting tube 42. During priming, by driving the liquid discharge pump 71 in a state in which the second electromagnetic valve 72 is closed and the third electromagnetic valve 73 is opened, suction of the blood circuit 21 is performed through the connecting flow path 40 that is connected on the upstream side of the liquid discharge pump 71. The second electromagnetic valve 72 is an example of a discharge-side on-off valve, and the third electromagnetic valve 73 is an example of an on-off valve.

The dialysate supply/discharge unit-side control unit 43 communicates with the control unit 24 of the extracorporeal circulation unit 11, and controls the liquid supply pump 61, the liquid discharge pump 71 and each of the electromagnetic valves 62, 72 and 73 according to commands from the control unit 24. The dialysate supply/discharge unit-side control unit 43 is realized by appropriately combining an arithmetic element such as CPU, a memory, software, interface and a communication unit, etc.

### Description of Control unit and its control

The control unit 24 and the control by this control unit 24 will now be described. The control unit 24 is realized by appropriately combining an arithmetic element such as CPU, a memory, software, interface and a communication unit, etc., receives a detection value of the air bubble detector 37 and controls the blood pump 33 and each of the clamps 34, 38 and 39. The control units 24 communicates with the dialysate supply/discharge unit-side control unit 43 and controls the liquid supply pump 61, the liquid discharge pump 71 and each of the electromagnetic valves 62, 72 and 73 through the dialysate supply/discharge unit-side control unit 43.

The control unit 24 executes a dialysis treatment operation by controlling the blood pump 33, the liquid supply pump 61 and the liquid discharge pump 71. During the dialysis treatment operation, the blood pump 33 is driven to circulate blood through the dialyzer 10 and the liquid supply pump 61 is driven to supply dialysate to the dialyzer 10, while the liquid discharge pump 71 is driven to discharge the effluent from the dialyzer 10. As a result, the dialysate is supplied to and discharged from the dialyzer 10 while circulating the blood of the patient C through the dialyzer 10, and the blood of the patient C is thereby purified.

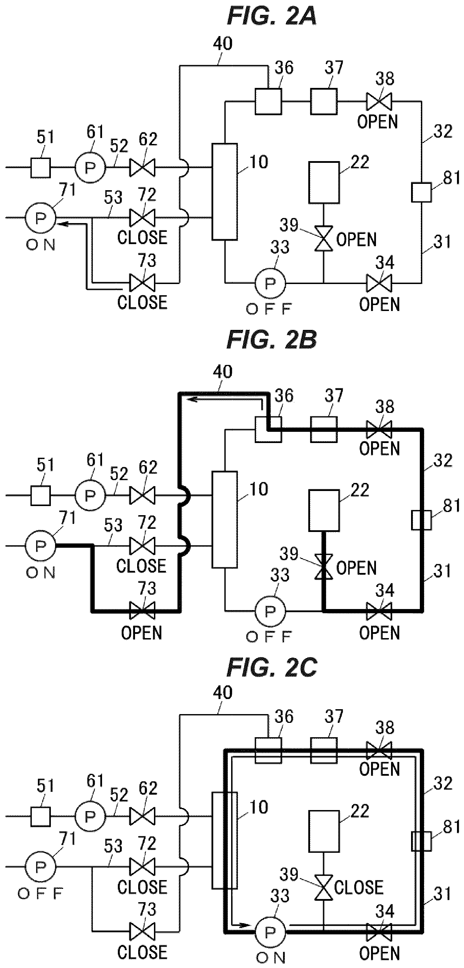

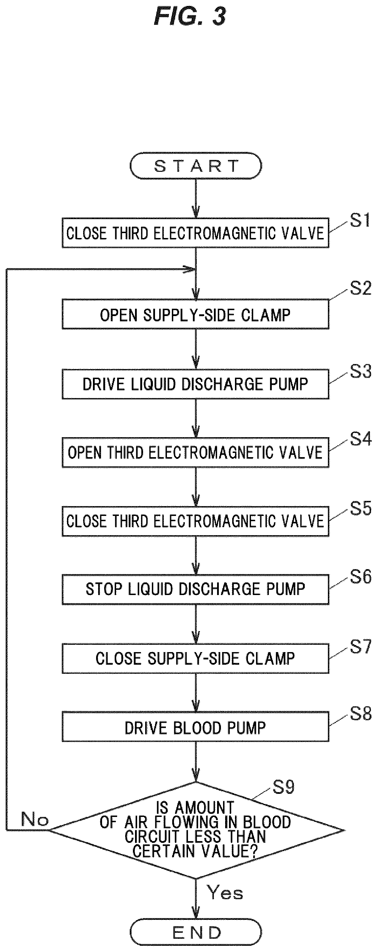

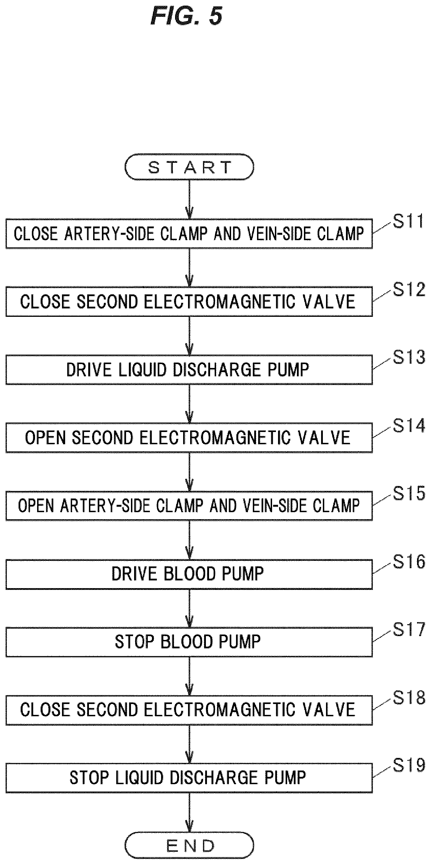

The control unit 24 also executes a priming operation and a replacement solution discharge operation by controlling the blood pump 33, the liquid discharge pump 71, each of the electromagnetic valves 62, 72, 73 and each of the clamps 34, 38, 39. Referring now to Figs. 2A to 5, the priming operation and the replacement solution discharge operation will be described.

### Description of Priming operation

The priming operation is an operation of filling the blood circuit 21 with saline solution before a hemodialysis operation. The priming operation is performed in a state in which the artery-side blood tube 31 and the vein-side blood tube 32 are in communication with each other by coupling their ends to each other with a coupler 81, as shown in Figs. 2A to 2C. In addition, the priming operation is performed in a state in which the second electromagnetic valve 72 is closed and the artery-side clamp 34 and the vein-side clamp 38 are opened. The control unit 24 executes the priming operation.

In the priming operation, first, the third electromagnetic valve 73 is closed (S1) and the supply-side clamp 39 is opened (S2), as shown in Figs. 2A to 3. After that, the liquid discharge pump 71 is driven (S3). That is, by driving the liquid discharge pump 71 in a state in which the blood circuit 21 and the effluent discharge flow path 53 are shut off by closing the third electromagnetic valve 73, negative pressure (pressure lower than the blood circuit 21) is created and accumulated in the tube from the liquid discharge pump 71 to the second electromagnetic valve 72 and the third electromagnetic valve 73 (in a predetermined region) (see Fig. 2A). This prevents the effluent in the effluent discharge flow path 53 from flowing back into the blood circuit 21 when the third electromagnetic valve 73 is opened. In this regard, whether or not negative pressure has been created in the tube from the liquid discharge pump 71 to the second electromagnetic valve 72 and the third electromagnetic valve 73 is determined, e.g., based on a detection value of a pressure detection unit provided on the tube from the liquid discharge pump 71 to the second electromagnetic valve 72 and the third electromagnetic valve 73. This pressure detection unit may be provided, e.g., on the effluent discharge flow path 53 between the liquid discharge pump 71 and the second electromagnetic valve 72 to also serve as a dialysate pressure sensor to detect (monitor) dialysate pressure during blood purification treatment, or another sensor used for the exclusive purpose or used in combination with other purposes can be provided instead.

After the negative pressure is created (accumulated) in the tube from the liquid discharge pump 71 to the third electromagnetic valve 73, the third electromagnetic valve 73 is opened while the liquid discharge pump 71 is still being driven (S4). Opening the third electromagnetic valve 73 produce a state in which the liquid discharge pump 71 is driven while the blood circuit 21 and the effluent discharge flow path 53 are connected in a manner that allows for liquid feeding through the connecting flow path 40. Thus, suction of the blood circuit 21 is performed through the connecting flow path 40 by the liquid discharge pump 71. That is, the blood circuit 21 is suctioned based on the accumulated pressure which is accumulated in S3. By suctioning the blood circuit 21 using the liquid discharge pump 71 and creating negative pressure in the blood circuit 21, the saline solution stored in the storage bag 22 flows into the artery-side blood tube 31. The saline solution flown into the blood circuit 21 passes through the vein-side blood tube 32 via the coupler 81 and reaches the air trap chamber 36. The saline solution then flows from the air trap chamber 36 into the effluent discharge flow path 53 through the connecting flow path 40 and reaches the liquid discharge pump 71. This causes the saline solution to fill the artery-side blood tube 31 and the vein-side blood tube 32 from a position connected to the storage bag 22 to the air trap chamber 36 and also to accumulate in the air trap chamber 36 (see Fig. 2B). That is, the saline solution in the blood circuit 21 is suctioned into the effluent discharge flow path 53 by driving the liquid discharge pump 71.

Once the flow path from the position connected to the storage bag 22 to the air trap chamber 36 is filled with the saline solution after a certain period of time has elapsed since the third electromagnetic valve 73 was opened, the third electromagnetic valve 73 is closed (S5) and the liquid discharge pump 71 is stopped (S6). After that, the supply-side clamp 39 is closed (S7), and the blood pump 33 is driven (driven in reverse) (S8). Driving the blood pump 33 causes the saline solution to flow and circulate in the blood circuit 21 while the air is partially trapped in the air trap chamber 36 (see Fig. 2C). Although the blood pump 33 is driven in reverse in the example shown in Fig. 2C, the blood pump 33 may be driven in forward.

After circulating the saline solution for a certain period of time, the amount of air flowing in the blood circuit 21 is detected by the air bubble detector 37 (S9). When it is determined as a result of the detection that the amount of air flowing in the blood circuit 21 is not less than a certain value (S9: No), the process returns to S2 and a series of actions from S2 to S8 is repeated. This gradually fills the blood circuit 21 with the saline solution and reduces the amount of air in the blood circuit 21. When there is no more air in the blood circuit 21 and it is determined that the amount of air flowing in the blood circuit 21 is less than the certain value (S9: Yes), this priming operation ends. The blood circuit 21 is thereby filled with the saline solution and the blood circuit 21 is primed. In this regard, since the saline solution also accumulates in the air trap chamber 36 when the saline solution is suctioned from the storage bag 22 (S4), the amount of the saline solution accumulating in the blood circuit 21 with one suction is large, hence, the number of repetitions of the above-mentioned series of actions (S2 to S8) can be small.

### Description of Replacement solution discharge operation

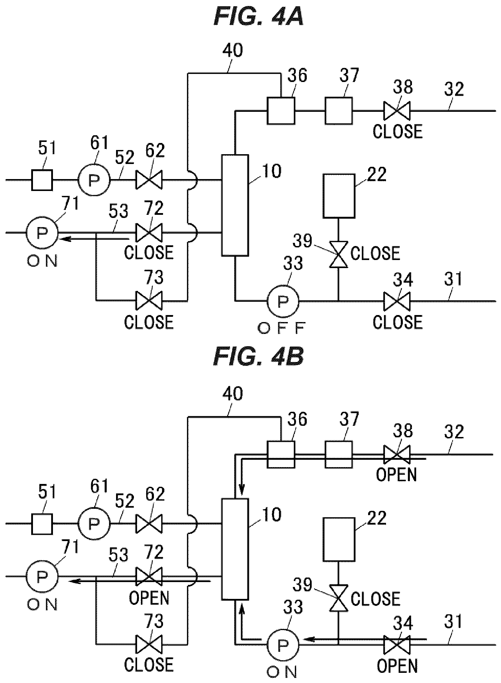

The replacement solution discharge operation is an operation performed after the blood purification device 1 returns the blood remaining in the blood circuit 21 to the patient C by filling the blood circuit 21 with a replacement solution (e.g., saline solution) after blood therapy, and it is an operation of discharging the replacement solution that is introduced to fill the blood circuit 21 during the blood return. In addition, the replacement solution discharge operation is performed in a state in which the artery-side blood tube 31 and the vein-side blood tube 32 are open to atmosphere after detaching their ends from the patient C, as shown in Figs. 4A and 4B. Furthermore, the replacement solution discharge operation is performed in a state in which the third electromagnetic valve 73 and the supply-side clamp 39 are closed. The control unit 24 executes the replacement solution discharge operation.

In the replacement solution discharge operation, first, the artery-side clamp 34 and the vein-side clamp 38 are closed (S11) and the second electromagnetic valve 72 is closed (S12), as shown in Figs. 4A to 5. After that, the liquid discharge pump 71 is driven (S13) (see Fig. 4A). That is, by driving the liquid discharge pump 71 in a state in which the second electromagnetic valve 72 is closed, negative pressure (pressure lower than the blood circuit 21) is created and accumulated in the tube from the liquid discharge pump 71 to the second electromagnetic valve 72 and the third electromagnetic valve 73 (in a predetermined region). This prevents the effluent in the effluent discharge flow path 53 from flowing back into the blood circuit 21 when the second electromagnetic valve 72 is opened. In this regard, whether or not negative pressure has been created in the tube from the liquid discharge pump 71 to the second electromagnetic valve 72 and the third electromagnetic valve 73 is determined, e.g., based on a detection value of a pressure detection unit provided on the tube from the liquid discharge pump 71 to the second electromagnetic valve 72 and the third electromagnetic valve 73. This pressure detection unit may be provided, e.g., on the effluent discharge flow path 53 between the liquid discharge pump 71 and the second electromagnetic valve 72 to also serve as a dialysate pressure sensor to detect (monitor) dialysate pressure during blood purification treatment, or another sensor used for the exclusive purpose or used in combination with other purposes can be provided instead.

After the negative pressure is created in the tube from the liquid discharge pump 71 to the second electromagnetic valve 72, the second electromagnetic valve 72 is opened (S14), the artery-side clamp 34 and the vein-side clamp 38 are opened (S15) and the blood pump 33 is driven (S16). Opening the second electromagnetic valve 72 and driving the blood pump 33 produce a state in which the liquid discharge pump 71 and the blood pump 33 are driven while the blood circuit 21 and the effluent discharge flow path 53 are connected in a manner that allows for liquid feeding through the dialyzer 10. Thus, the replacement solution in the blood circuit 21 is suctioned through the dialyzer 10 into the effluent discharge flow path 53 by the liquid discharge pump 71. That is, the replacement solution in the blood circuit 21 is suctioned based on the accumulated pressure which is accumulated in S13. In particular, by driving the liquid discharge pump 71 and the blood pump 33, pressure of the liquid discharge pump 71 introduces air into the vein-side blood tube 32 and at the same time causes the replacement solution in the vein-side blood tube 32 to flow and to be discharged into the effluent discharge flow path 53 through dialyzer 10, while pressure of the blood pump 33 introduces air into the artery-side blood tube 31 and at the same time causes the replacement solution in the artery-side blood tube 31 to flow and to be discharged into the effluent discharge flow path 53 through dialyzer 10 (see Fig. 4B). The replacement solution in the blood circuit 21 is thereby discharged through the effluent discharge flow path 53. In this regard, if the flow rate of the liquid discharge pump 71 and the flow rate of the blood pump 33 are equal, only the replacement solution in the artery-side blood tube 31 is discharged and the replacement solution in the vein-side blood tube 32 is not discharged. Therefore, the target flow rate of the liquid discharge pump 71 is set higher than the target flow rate of the blood pump 33 by the amount required for discharging the replacement solution from the vein-side blood tube 32.

When the discharge of the replacement solution is finished, the blood pump 33 is stopped (S17), the second electromagnetic valve 72 is closed (S18), and the liquid discharge pump 71 is stopped (S19). This replacement solution discharge operation thereby ends.

### Functions and Effects of the embodiment

In the configuration of the embodiment described above, since suction of the blood circuit 21 for priming or discharge of the replacement solution is performed using the liquid discharge pump 71, the configuration is simple and also the priming or the discharge of the replacement solution can be performed quickly. That is, by configuring such that the liquid discharge pump 71, which causes pressure for discharge from the dialyzer 10 to be applied to the effluent, can be driven independently from the liquid supply pump 61 and is used for suction of the blood circuit 21, it is possible to suction the blood circuit 21 at high pressure. This allows the priming or the discharge of the replacement solution to be performed more quickly (e.g., at a rate about 30 times faster than when using a water removal pump) and also eliminates the need to separately provide a high-pressure pump, hence, the blood purification device 1 can have a simple configuration.

### Modification

Although the embodiment of the invention has been described, the invention according to claims is not to be limited to the embodiment described above. Further, please note that not all combinations of the features described in the embodiment are necessary to solve the problem of the invention.

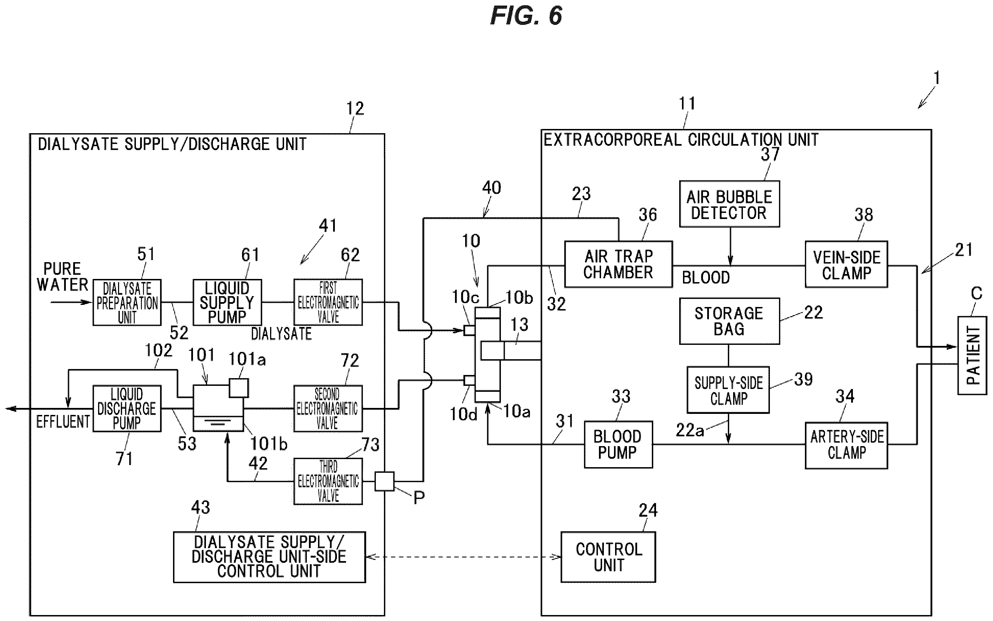

For example, as shown in Fig. 6, the configuration may be such that an air bubble trap 101 (an example of the air bubble removal unit), which is arranged on the effluent discharge flow path 53 on the upstream side of the liquid discharge pump 71 and removes air bubbles in the effluent, is further included and the dialysate circuit 41 side of the connecting flow path 40 (the dialysate circuit-side connecting tube 42) is connected to the air bubble trap 101. In such a case, the air bubble trap 101 includes, e.g., an air bubble detection sensor 101a to detect air bubbles in the effluent, and a degassing chamber 101b. Then, it is configured such that, e.g., the dialysate circuit 41 further has a discharge flow path 102 connected to the effluent discharge flow path 53 to discharge a liquid while bypassing the liquid discharge pump 71, the amount of air in the degassing chamber 101b is detected by the air bubble detection sensor 101a, and when the amount of air in the degassing chamber 101b becomes not less than a predetermined amount, the air in the degassing chamber 101b is discharged through the discharge flow path 102. With such a configuration, it is possible to prevent the air from entering the liquid discharge pump 71 during the priming operation. A decrease in the discharge force of the liquid discharge pump 71 due to air entrapment can thereby be prevented. That is, this configuration allows for use of a small liquid discharge pump 71 whose discharge force decreases at the event of air entrapment, hence, the liquid discharge pump 71 can be reduced in size. In addition, since the air bubble trap 101 arranged on the effluent discharge flow path 53 on the upstream side of the liquid discharge pump 71 is also used to remove air during the priming operation, a single air bubble trap 101 can be used to remove air from the effluent during the dialysis treatment operation and also remove air from the liquid discharged during the priming operation, thereby allowing the blood purification device 1 to have a simple configuration.

In addition, in the configuration shown in Fig. 6, by arranging the air bubble detection sensor 101a on the effluent discharge flow path 53 on the upstream side of the liquid discharge pump 71, it is possible to prevent air from entering the liquid discharge pump 71 also during the replacement solution discharge operation. Furthermore, at the time of discharging the replacement solution in the blood circuit 21 into the effluent discharge flow path 53 (at the time of discharging the replacement solution in the replacement solution discharge operation) in the configuration shown in Fig. 6, by discharging the replacement solution through the discharge flow path 102 when air bubbles in the replacement solution are detected by the air bubble detection sensor 101a, the replacement solution can be discharged with no air entering the liquid discharge pump 71.

In addition, although discharge of the replacement solution during the replacement solution discharge operation is performed through the dialyzer 10 in the configuration of the embodiment described above, it is not limited thereto. That is, discharge of the replacement solution during the replacement solution discharge operation may be performed in a state in which the blood circuit 21 and the effluent discharge flow path 53 are connected through the connecting flow path 40 by opening the third electromagnetic valve 73 while closing the second electromagnetic valve 72. In other words, discharge of the replacement solution during the replacement solution discharge operation may be performed by driving the liquid discharge pump 71 in the state in which the blood circuit 21 and the effluent discharge flow path 53 are connected through the connecting flow path 40. In such a case, the liquid discharge pump 71 is driven in the state in which the third electromagnetic valve 73 is closed, negative pressure is created and accumulated in the tube from the liquid discharge pump 71 to the third electromagnetic valve 73 (in a predetermined region), and the third electromagnetic valve 73 is then opened to discharge the replacement solution, in the same manner as the priming operation.

### Summary of the embodiment

Technical ideas understood from the embodiment will be described below citing the reference signs, etc., used for the embodiment. However, each reference sign, etc., described below is not intended to limit the constituent elements in the claims to the members, etc., specifically described in the embodiment.
(1) A blood purification device 1 to purify blood of a patient C through a blood purifier 10 comprising a blood purification membrane therein, the blood purification device 1 comprising: a blood circuit 21 to circulate the blood through the blood purifier 10; a dialysate supply flow path 52 to supply dialysate to the blood purifier 10; an effluent discharge flow path 53 to discharge effluent from the blood purifier 10; a liquid supply pump 61 arranged on the dialysate supply flow path 52 to cause pressure for supply to the blood purifier 10 to be applied to the dialysate; and a liquid discharge pump 71 that is arranged on the effluent discharge flow path 53, is provided separately and can be driven independently from the liquid supply pump 61, and causes pressure for discharge from the blood purifier 10 to be applied to the effluent, wherein a liquid in the blood circuit 21 is suctioned into the effluent discharge flow path 53 by driving the liquid discharge pump 71 in a state in which the blood circuit 21 and the effluent discharge flow path 53 are connected in a manner that allows for liquid feeding through a connecting flow path 40 connecting the blood circuit 21 to the effluent discharge flow path 53 while bypassing the blood purifier 10, or through the blood purifier 10.
   It is thereby possible to have a simple configuration and also perform priming or discharge of the replacement solution quickly.
(2) The blood purification device 1 as defined by (1), further comprising: a storage unit 22 that is connected to the blood circuit 21 through a supply tube 22a and stores a priming solution; and the connecting flow path 40, wherein by driving the liquid discharge pump 71 in a state in which the blood circuit 21 and the effluent discharge flow path 53 are connected through the connecting flow path 40, the priming solution from the storage unit 22 fills the blood circuit 21 and the blood circuit 21 is primed.
   It is thereby possible to have a simple configuration and also perform priming quickly.
(3) The blood purification device 1 as defined by (1) or (2), further comprising: an air bubble removal unit 101 that is arranged on the effluent discharge flow path 53 on the upstream side of the liquid discharge pump 71 and removes air bubbles present in the effluent, wherein the connecting flow path 40 is connected to the air bubble removal unit 101.
   It is thereby possible to prevent air from entering the liquid discharge pump.
(4) The blood purification device 1 as defined by (1) or (2), wherein an on-off valve 73 to open and close the connecting flow path 40 is arranged on the connecting flow path 40, and wherein after accumulating pressure in a predetermined region of the effluent discharge flow path 53 and the connecting flow path 40 by driving the liquid discharge pump 71 in a state in which the blood circuit 21 and the effluent discharge flow path 53 are shut off by closing the on-off valve 73, the on-off valve 73 is opened and a liquid in the blood circuit 21 is thereby suctioned into the effluent discharge flow path 53 based on the accumulated pressure.
   It is thereby possible to prevent the effluent in the effluent discharge flow path from flowing back into the blood circuit.
(5) The blood purification device 1 as defined by any one of (1) to (3), wherein after blood purification treatment, the blood remaining in the blood circuit 21 is returned to a patient C by filling the blood circuit 21 with a replacement solution, and wherein by driving the liquid discharge pump 71 in a state in which the blood circuit 21 and the effluent discharge flow path 53 are connected through the blood purifier 10, the replacement solution is discharged into the effluent discharge flow path 53 while introducing air into the blood circuit 21.
   It is thereby possible to have a simple configuration and also perform discharge of the replacement solution quickly.
(6) The blood purification device 1 defined by (5), wherein a discharge-side on-off valve 72 to open and close the effluent discharge flow path 53 is arranged on the effluent discharge flow path 53 on the upstream side of the liquid discharge pump 71, and wherein after accumulating pressure in a predetermined region of the effluent discharge flow path 53 by driving the liquid discharge pump 71 in a state in which the discharge-side on-off valve 72 is closed, the discharge-side on-off valve 72 is opened and a liquid in the blood circuit 21 is thereby suctioned into the effluent discharge flow path 53 based on the accumulated pressure.
   It is thereby possible to prevent the effluent in the effluent discharge flow path from flowing back into the blood circuit.
(7) The blood purification device 1 as defined by (5) or (6), further comprising: an air bubble detection sensor 101a that is arranged on the effluent discharge flow path 53 on the upstream side of the liquid discharge pump 71 and detects air bubbles in the effluent. It is thereby possible to prevent air from entering the liquid discharge pump.
(8) The blood purification device 1 as defined by (7), further comprising: a discharge flow path 102 connected to the effluent discharge flow path 53 to discharge a liquid while bypassing the liquid discharge pump 71, wherein at the time of discharging the replacement solution in the blood circuit 21 into the effluent discharge flow path 53, the replacement solution is discharged through the discharge flow path 102 when air bubbles in the replacement solution are detected by the air bubble detection sensor 101a. It is thereby possible to discharge the replacement solution with no air entering the liquid discharge pump during the replacement solution discharge operation.

### REFERENCE SIGNS LIST

1: blood purification device, 10: dialyzer, 21: blood circuit, 22: storage bag, 22a: supply tube, 40: connecting flow path, 52: dialysate supply flow path, 53: effluent discharge flow path, 61: liquid supply pump, 71: liquid discharge pump, 72: second electromagnetic valve, 73: third electromagnetic valve, 101: air bubble removal unit, 101a: air bubble detection sensor, 102: discharge flow path, C: patient

## Claims

1. A blood purification device to purify blood of a patient through a blood purifier comprising a blood purification membrane therein, the blood purification device comprising:
a blood circuit to circulate the blood through the blood purifier;
a dialysate supply flow path to supply dialysate to the blood purifier;
an effluent discharge flow path to discharge effluent from the blood purifier;
a liquid supply pump arranged on the dialysate supply flow path to cause pressure for supply to the blood purifier to be applied to the dialysate; and
a liquid discharge pump that is arranged on the effluent discharge flow path, is provided separately and can be driven independently from the liquid supply pump, and causes pressure for discharge from the blood purifier to be applied to the effluent,
wherein a liquid in the blood circuit is suctioned into the effluent discharge flow path by driving the liquid discharge pump in a state in which the blood circuit and the effluent discharge flow path are connected in a manner that allows for liquid feeding through a connecting flow path connecting the blood circuit to the effluent discharge flow path while bypassing the blood purifier, or through the blood purifier.

2. The blood purification device according to claim 1, further comprising:
a storage unit that is connected to the blood circuit through a supply tube and stores a priming solution; and
the connecting flow path,
wherein by driving the liquid discharge pump in a state in which the blood circuit and the effluent discharge flow path are connected through the connecting flow path, the priming solution from the storage unit fills the blood circuit and the blood circuit is primed.

3. The blood purification device according to claim 1 or 2, further comprising:
an air bubble removal unit that is arranged on the effluent discharge flow path on the upstream side of the liquid discharge pump and removes air bubbles present in the effluent,
wherein the connecting flow path is connected to the air bubble removal unit.

4. The blood purification device according to claim 1 or 2, wherein an on-off valve to open and close the connecting flow path is arranged on the connecting flow path, and wherein after accumulating pressure in a predetermined region of the effluent discharge flow path and the connecting flow path by driving the liquid discharge pump in a state in which the blood circuit and the effluent discharge flow path are shut off by closing the on-off valve, the on-off valve is opened and a liquid in the blood circuit is thereby suctioned into the effluent discharge flow path based on the accumulated pressure.

5. The blood purification device according to claim 1, wherein after blood purification treatment, the blood remaining in the blood circuit is returned to a patient by filling the blood circuit with a replacement solution, and wherein by driving the liquid discharge pump in a state in which the blood circuit and the effluent discharge flow path are connected through the blood purifier, the replacement solution is discharged into the effluent discharge flow path while introducing air into the blood circuit.

6. The blood purification device according to claim 5, wherein a discharge-side on-off valve to open and close the effluent discharge flow path is arranged on the effluent discharge flow path on the upstream side of the liquid discharge pump, and wherein after accumulating pressure in a predetermined region of the effluent discharge flow path by driving the liquid discharge pump in a state in which the discharge-side on-off valve is closed, the discharge-side on-off valve is opened and a liquid in the blood circuit is thereby suctioned into the effluent discharge flow path based on the accumulated pressure.

7. The blood purification device according to claim 5 or 6, further comprising:
an air bubble detection sensor that is arranged on the effluent discharge flow path on the upstream side of the liquid discharge pump and detects air bubbles in the effluent.

8. The blood purification device according to claim 7, further comprising:
a discharge flow path connected to the effluent discharge flow path to discharge a liquid while bypassing the liquid discharge pump,
wherein at the time of discharging the replacement solution in the blood circuit into the effluent discharge flow path, the replacement solution is discharged through the discharge flow path when air bubbles in the replacement solution are detected by the air bubble detection sensor.
